# FASCICULE DE BREVET EUROPEEN

(11) **EP 4 054 455 B1**
(45) Date de publication et mention de la délivrance du brevet: **03.01.2024**
(21) Numéro de dépôt: 20817464.9
(22) Date de dépôt: 05.11.2020
(51) Int. Cl.: A61B 17/86, A61B 17/70

(54) **VIS D'ANCRAGE TRANS-PÉDICULAIRE À STABILITÉ SECONDAIRE RENFORCÉE**
TRANSPEDIKULÄRE VERANKERUNGSSCHRAUBE MIT VERSTÄRKTER SEKUNDÄRSTABILITÄT
TRANSPEDICULAR ANCHORING SCREW WITH REINFORCED SECONDARY STABILITY

(30) Priorité: 07.11.2019 FR 1912517
(43) Date de publication de la demande: 14.09.2022
(73) Titulaire: Spinedust, 13008 Marseille (FR)
(72) Inventeur: GRAZIANI, Noël, 13004 MARSEILLE (FR); LEVRIER, Olivier, 13008 Marseille (FR)
(74) Mandataire: Germain Maureau
(86) Numéro de dépôt international: PCT/FR2020/052000
(87) Numéro de publication internationale: WO 2021/089944

(56) Documents cités:
- EP-A1- 3 329 871

## Description

La présente invention concerne une vis d'ancrage trans-pédiculaire, adaptée pour un ancrage dans le pédicule d'une vertèbre d'un corps humain.

Dans le domaine chirurgical, il est connu d'utiliser des implants rachidiens fixés sur une ou plusieurs vertèbres au moyen d'une ou plusieurs vis d'ancrage.

Par exemple, il est connu de fixer sur deux vertèbres successives des implants d'arthrodèse vertébrale permettant une fusion de ces deux vertèbres et une stabilisation générale du rachis.

De tels implants sont généralement fixés sur les vertèbres au moyen de vis d'ancrage spécialement adaptées pour coopérer avec lesdits implants et pénétrant dans la structure osseuse des vertèbres.

Chaque vis d'ancrage permet ainsi d'assurer un ancrage fiable d'un implant sur une vertèbre caractérisé par :
- une stabilité primaire, résultant des efforts mécaniques exercés entre l'os de la vertèbre et la vis d'ancrage dès l'insertion de celle-ci dans la vertèbre, et
- une stabilité secondaire, résultant d'un processus d'ostéo-intégration, c'est-à-dire résultant de la régénération osseuse et du remodelage osseux se produisant autour de la vis d'ancrage après l'insertion de celle-ci dans la vertèbre.

La qualité de la stabilité primaire et de la qualité secondaire procurées par une vis d'ancrage dépend ainsi de plusieurs paramètres différents, et en particulier de la géométrie de la vis d'ancrage, de la qualité de la structure osseuse de la vertèbre dans laquelle elle est insérée, ou encore de la position d'insertion de la vis d'ancrage par rapport à la vertèbre.

Plus précisément, une vis d'ancrage peut être insérée dans une vertèbre par un abord antérieur, la vis d'ancrage traversant alors uniquement le corps vertébral de ladite vertèbre, ou par un abord postérieur, la vis d'ancrage traversant alors d'abord un pédicule puis le corps vertébral de ladite vertèbre.

Dans le cas d'un abord postérieur, la vis d'ancrage, une fois implantée, traverse trois types de structures osseuses de la vertèbre, chacune présentant des propriétés mécaniques différentes :
- la paroi corticale du pédicule, formée d'un os compact et dur,
- une portion centrale du pédicule, formée d'un os spongieux assez dense, et
- un corps spongieux du corps vertébral, formé d'un os moins dense et plus fragile.

Il est également à noter qu'il est envisageable que la vis d'ancrage puisse traverser, à l'extrémité de sa tige, la paroi corticale du corps vertébral, formée d'un os compact et dur.

Chacune de ces structures osseuse participe donc à la stabilité primaire de la vis d'ancrage de manière inégale, les parois corticales du pédicule et du corps vertébral offrant un ancrage fort et solide tandis que le corps spongieux du corps vertébral procure une stabilité primaire beaucoup plus faible, voire inexistante.

Il est ainsi usuel d'adapter la géométrie de la vis d'ancrage pour améliorer la stabilité primaire de celle-ci, en augmentant la taille des surfaces de contact entre cette dernière et les parois corticales du pédicule et du corps vertébral.

Il est également déjà connu de l'état de la technique de modifier la structure de la vis d'ancrage dans le but d'améliorer sa stabilité secondaire.

Par exemple, les documents WO 14184463 et EP1682021 décrivent chacun une vis d'ancrage présentant un filet hélicoïdal comportant plusieurs encoches latérales de mêmes taille et forme : une fois une telle vis implantée dans une vertèbre, l'os à proximité de la vis d'ancrage est amené à se régénérer et une croissance osseuse a lieu dans les encoches, comblant celles-ci et augmentant ainsi la surface de contact avec la vis d'ancrage.

Cependant, du fait de la faible taille de ces encoches, celles-ci ne permettent d'améliorer la stabilité secondaire de la vis d'ancrage que de manière très marginale. De plus, de telles vis d'ancrage présentant un unique type d'encoches font face à une incompatibilité :
- dans le cas où les encoches sont de faible taille, elles ne permettent pas de profiter pleinement de la stabilité secondaire potentiellement procurée par le corps spongieux du corps vertébral, et
- dans le cas où les encoches sont de grande taille, elles dégradent la stabilité primaire de la vis d'ancrage, du fait de la réduction de la taille des surfaces de contact entre celle-ci et les parois corticales du pédicule.

Le document EP3329871 décrit quant à lui une vis d'ancrage dont le filet comporte une première portion distale (à proximité de la pointe de la vis d'ancrage) et une portion proximale (à proximité de la tête de la vis d'ancrage) de structure différente, le filetage présentant dans la portion distale des crénelures absentes de la portion proximale. Cependant, cette vis d'ancrage présente deux inconvénients majeurs :
- du fait de l'absence de crénelures dans la portion proximale, cette vis d'ancrage ne profite pas de la stabilité secondaire potentiellement procurée par la paroi corticale et la portion centrale du pédicule ; et
- du fait de la proximité immédiate entre la pointe de la vis d'ancrage et le filetage crénelé, celle-ci risque d'endommager, pendant son implantation, les structures osseuses avoisinantes, détériorant ainsi sa stabilité primaire et augmentant les risques vasculaires et neurologiques encourus par un patient, notamment un patient ostéopénique.

La présente invention a ainsi pour but de résoudre en tout ou partie les inconvénients précités, en proposant une vis d'ancrage trans-pédiculaire présentant une stabilité secondaire renforcée, sans dégrader sa stabilité primaire.

Un autre objectif de l'invention est de proposer une vis d'ancrage trans-pédiculaire dont l'implantation dans une vertèbre n'est pas délétère, c'est à dire n'est pas de nature à endommager la structure osseuse de ladite vertèbre.

Encore un autre but de l'invention est de proposer une vis d'ancrage trans-pédiculaire qui puisse être implantée dans une vertèbre à l'aide d'outils standards et qui reste d'utilisation simple et de fabrication peu onéreuse.

A cet effet, elle propose une vis d'ancrage trans-pédiculaire, adaptée pour un ancrage dans un pédicule d'une vertèbre, comportant un corps de vis s'étendant selon un axe de vis entre une extrémité proximale et une extrémité distale, ledit corps de vis présentant un premier filet et un deuxième filet, chacun desdits premier filet et deuxième filet étant de forme hélicoïdale avec un même pas de vis, s'étendant entre ladite extrémité proximale et ladite extrémité distale et présentant un bord externe de forme convexe,

ladite vis d'ancrage trans-pédiculaire étant remarquable en ce qu'au moins ledit premier filet présente, depuis l'extrémité proximale vers l'extrémité distale, au moins :
- une partie proximale, dans laquelle le bord externe dudit premier filet présente une succession de portions encochées proximales, chacune définie par une encoche proximale ménagée dans ledit bord externe et s'inscrivant dans un secteur angulaire proximal encoché, où deux portions encochées proximales successives sont séparées l'une de l'autre par une portion de séparation proximale, dans laquelle ledit bord externe n'est pas encoché et s'inscrivant dans un secteur angulaire proximal de séparation, et dans laquelle, sur une longueur du premier filet correspondant à un pas de vis, un rapport entre la somme des mesures de tous les secteurs angulaires proximaux de séparation et la somme des mesures de tous les secteurs angulaires proximaux encochés est compris entre 50% et 150% ; et
- une partie distale, dans laquelle le bord externe dudit premier filet présente une succession de portions encochées distales, chacune définie par une encoche distale ménagée dans ledit bord externe et s'inscrivant dans un secteur angulaire distal encoché, où deux portions encochées distales successives sont séparées par une portion de séparation distale, dans laquelle ledit bord externe n'est pas encoché et s'inscrivant dans un secteur angulaire distal de séparation, et dans laquelle, sur une longueur du premier filet correspondant à un pas de vis, un rapport entre la somme des mesures de tous les secteurs angulaires distaux de séparation et la somme des mesures de tous les secteurs angulaires distaux encochés est compris entre 0% et 10%.

L'invention propose ainsi une vis d'ancrage trans-pédiculaire à double filet hélicoïdal, la géométrie de l'un de ses deux filets étant spécialement adaptée pour améliorer la stabilité secondaire de la vis d'ancrage trans-pédiculaire selon l'invention, sans dégrader sa stabilité primaire.

En effet, un premierfilet de la vis d'ancrage trans-pédiculaire selon l'invention présente un bord externe généralement convexe, par exemple une forme hélicoïdale circulaire, dans lequel sont ménagées des encoches de taille et de forme variables.

Ces encoches, une fois la vis d'ancrage trans-pédiculaire implantée dans une vertèbre, favorisent la régénération osseuse de ladite vertèbre et sont destinées à être au moins partiellement comblées par des structures osseuses à l'issue d'un certain intervalle temporel, améliorant ainsi la stabilité secondaire de la vis d'ancrage trans-pédiculaire du fait de l'augmentation de la taille des surfaces de contact entre ladite vis d'ancrage trans-pédiculaire et ladite vertèbre.

Plus particulièrement, le premier filet de la vis d'ancrage trans-pédiculaire présente deux parties de géométrie distinctes :
- une partie proximale, destinée à être implantée dans un pédicule d'une vertèbre, dans laquelle le premierfilet présente une succession d'encoches proximales (définissant des portions encochées proximales du bord externe dudit premier filet) séparées les unes des autres par des portions de séparation proximales, lesdites portions de séparation proximales étant non encochées et présentant une largeur, mesurée le long du bord externe, importante ; et
- une partie distale, destinée à être implantée dans le corps spongieux du corps vertébral de la même vertèbre, dans laquelle le premier filet présente une succession d'encoches distales (définissant des portions encochées distales du bord externe dudit premier filet) séparées les unes des autres par des portions de séparation distales, lesdites portions de séparation distales étant non encochées et présentant une largeur, mesurée le long du bord externe, faible voire nulle. Le premier filet présente donc, dans sa partie distale, une succession d'encoches distales, de forme généralement concave et très proches les unes des autres : ce premier filet a donc une forme « en étoile », les portions de séparation distales (disposées entre les encoches distales) constituant des saillies radiales en forme de pointe acérée.

Cette structure particulière du filet distal présente deux avantages principaux :
- du fait de la grande surface occupée par les encoches distales, celles-ci favorisent une régénération osseuse importante de la vertèbre autour de la partie distale du premier filet, améliorant fortement la stabilité secondaire de la vis d'ancrage trans-pédiculaire ; et
- pendant l'insertion de la vis d'ancrage trans-pédiculaire dans la vertèbre, les saillies radiales formées par les encoches distales créent à leur périphérie une zone de remodelage osseux favorisant également la stabilité secondaire de la vis d'ancrage trans-pédiculaire.

Les encoches proximales, ménagées dans la partie proximale du premier filet, ont également pour but d'augmenter la stabilité secondaire de la vis d'ancrage trans-pédiculaire, en favorisant la régénération osseuse du pédicule dans lequel cette partie proximale est implantée.

Cependant, la surface occupée par ces encoches proximales est beaucoup moins importante que celle occupée par les encoches distales (du fait de la plus grande largeur des portions de séparation proximales), afin de préserver la stabilité primaire de la vis d'ancrage trans-pédiculaire.

En effet, comme précédemment évoqué, la stabilité primaire de la vis d'ancrage trans-pédiculaire résulte principalement des efforts mécaniques exercés sur celle-ci par les structures osseuses de la vertèbre dans laquelle elle est implantée, au niveau de la paroi corticale et de la portion centrale du pédicule : la stabilité primaire de la vis d'ancrage trans-pédiculaire dépend ainsi principalement de la taille de la surface de contact entre la partie proximale du premier filet et les structures osseuses dudit pédicule.

Afin de préserver une telle surface de contact, il est donc nécessaire de restreindre la taille des encoches proximales et d'augmenter celle des portions de séparation proximales.

En revanche, le corps spongieux dans lequel la partie distale est destinée à être implantée ne contribue que très faiblement à la stabilité primaire de la vis d'ancrage trans-pédiculaire : l'augmentation de la surface occupée par les encoches distales et la réduction de celle des portions de séparation distales ne fait diminuer la stabilité primaire de la vis d'ancrage trans-pédiculaire qu'à la marge.

Ainsi, en proposant un premier filet présentant des encoches proximales et distales dont la taille et/ou la forme varie en fonction de leur zone d'implantation (corps spongieux du corps vertébral ou os pédiculaire plus dense), la vis d'ancrage trans-pédiculaire selon l'invention présente bien une stabilité secondaire renforcée, sans que sa stabilité primaire ne soit sensiblement modifiée.

On notera que, au sens de l'invention, la largeur des portions encochées distales, des portions encochées proximales, des portions de séparation distales et des portions de séparation proximales est mesurée le long du bord externe, et est caractérisée de manière équivalente par les secteurs angulaires, distaux ou proximaux, encochés ou de séparation (observés depuis l'axe de vis) dans lesquels chacune de ces portions s'inscrit. En particulier, les portions encochées proximales et distales sont chacune définies respectivement par une encoche proximale ou distale : leur « largeur » correspond au secteur angulaire proximale ou distale encoché sous lequel ladite encoche est vue depuis l'axe de vis.

Au sens de l'invention, chaque encoche proximale (respectivement distale) est ainsi contiguë à une portion de séparation proximale (respectivement distale), et inversement.

Dans la partie proximale, sur une longueur du premier filet correspondant à un pas de vis (c'est-à-dire l'intervalle dans lequel le premier filet effectue un enroulement d'un tour complet à 360° autour du corps de vis), le rapport entre la somme des mesures de tous les secteurs angulaires proximaux de séparation et la somme des mesures de tous les secteurs angulaires proximaux encochés est compris entre 50% et 150% : en d'autres termes, les secteurs angulaires proximaux encochés occupent une portion du premier filet substantiellement équivalente à celle occupée par les secteurs angulaires proximaux de séparation (l'une de ces portions respectives ne pouvant être plus de deux fois supérieure à l'autre).

De la sorte, la partie proximale confère à la vis d'ancrage trans-pédiculaire une bonne stabilité primaire car celle-ci reste en contact (par le biais des portions de séparation proximales) avec l'os cortical du pédicule dans lequel ladite vis d'ancrage trans-pédiculaire est implantée.

A l'inverse, dans la partie distale, sur une longueur du premier filet correspondant à un pas de vis, le rapport entre la somme des mesures de tous les secteurs angulaires distaux de séparation et la somme des mesures de tous les secteurs angulaires distaux encochés est compris entre 0% et 10% : en d'autres termes, les secteurs angulaires distaux encochés occupent une portion du premier filet très largement supérieure (au moins dix fois supérieure) à celle occupée par les secteurs angulaires distaux de séparation.

De la sorte, la partie distale confère à la vis d'ancrage trans-pédiculaire une très bonne stabilité secondaire mais une stabilité primaire moindre.

On notera bien que, au sens de l'invention, le rapport entre la somme des mesures de tous les secteurs angulaires de séparation et la somme des mesures de tous les secteurs angulaires encochés correspond au résultat d'une opération de division de la somme des mesures de tous les secteurs angulaires de séparation par la somme des mesures de tous les secteurs angulaires encochés.

Selon une caractéristique, dans la portion proximale, la mesure de chaque secteur angulaire proximal de séparation est supérieure à 15°.

Selon une autre caractéristique, dans la portion distale, la mesure de chaque secteur angulaire distal de séparation est inférieure à 5°.

La vis d'ancrage trans-pédiculaire selon l'invention présente également un deuxième filet, entrelacé avec le premierfilet, présentant une forme hélicoïdale et un pas similaire à celui-ci.

Dans un premier mode de réalisation, le bord externe de ce deuxième filet est dépourvu d'encoches distales ou proximales.

De la sorte, la vis d'ancrage trans-pédiculaire présente, selon l'axe de vis, une alternance entre le premier filet, comportant des encoches proximales et distales, et le deuxième filet, dépourvu d'encoches et présentant un profil standard : la présence du deuxième filet permet en particulier d'augmenter la surface de contact externe de la vis d'ancrage trans-pédiculaire à proximité de la partie distale du premier filet.

Cette alternance des deux filets permet ainsi notamment :
- d'augmenter la stabilité primaire de la vis d'ancrage trans-pédiculaire, du fait de l'augmentation de la taille de la surface de contact entre celle-ci et les structures osseuses d'une vertèbre dans laquelle elle est implantée, et
- de « protéger » lesdites structures osseuses des éventuelles dégradations provoquées par les saillies radiales de la partie distale du premier filet au cours de l'implantation de la vis d'ancrage trans-pédiculaire.

Dans une variante, le deuxième filet présente, depuis l'extrémité proximale vers l'extrémité distale, au moins :
- une partie proximale, dans laquelle le bord externe dudit deuxième filet présente une succession de portions encochées proximales, chacune définie par une encoche proximale ménagée dans ledit bord externe et s'inscrivant dans un secteur angulaire proximal encoché, où deux portions encochées proximales successives sont séparées l'une de l'autre par une portion de séparation proximale, dans laquelle ledit bord externe n'est pas encoché et s'inscrivant dans un secteur angulaire proximal de séparation, et dans laquelle, sur une longueur du deuxième filet correspondant à un pas de vis, un rapport entre la somme des mesures de tous les secteurs angulaires proximaux de séparation et la somme des mesures de tous les secteurs angulaires proximaux encochés est compris entre 50% et 150% ; et
- une partie distale, dans laquelle le bord externe dudit deuxième filet présente une succession de portions encochées distales, chacune définie par une encoche distale ménagée dans ledit bord externe et s'inscrivant dans un secteur angulaire distal encoché, où deux portions encochées distales successives sont séparées par une portion de séparation distale, dans laquelle ledit bord externe n'est pas encoché et s'inscrivant dans un secteur angulaire distal de séparation, et dans laquelle, sur une longueur du deuxième filet correspondant à un pas de vis, un rapport entre la somme des mesures de tous les secteurs angulaires distaux de séparation et la somme des mesures de tous les secteurs angulaires distaux encochés est compris entre 0% et 10%. En d'autres termes, il est envisagé que, dans un deuxième mode de réalisation de l'invention, le deuxième filet présente le même type d'encoche et en même proportion que le premier filet. Avantageusement, la vis d'ancrage trans-pédiculaire selon l'invention présente une tête de vis, adaptée pour coopérer avec un implant chirurgical destiné à être fixé sur une vertèbre, disposée à l'extrémité proximale et une pointe d'attaque, adaptée pour favoriser la pénétration de ladite vis d'ancrage dans ladite vertèbre, disposée à l'extrémité distale.

Il est envisageable que la pointe d'attaque, à l'issue d'une implantation de la vis d'ancrage trans-pédiculaire selon l'invention dans une vertèbre, se trouve ancrée dans la paroi corticale du corps de vertèbre, améliorant ainsi la stabilité primaire de ladite vis d'ancrage trans-pédiculaire.

On notera que, au cours d'une implantation de la vis d'ancrage trans-pédiculaire selon l'invention dans une vertèbre, la partie distale du premier filet traverse le pédicule de ladite vertèbre, avant de pénétrer dans le corps spongieux dudit corps de vertèbre.

Dans une variante, la partie distale du premier filet s'étend jusqu'à la pointe d'attaque. Il est à noter que les encoches distales peuvent être prévues sur tout ou partie de la partie distale.

De nombreux modes de réalisation concernant le nombre, la taille, la forme ou la répartition des encoches proximales et distales le long du premier filet sont envisageables, dont quelques-uns sont décrits, à titre non limitatif, ci-après.

Dans un mode de réalisation, chaque encoche proximale présente une forme identique et chaque portion de séparation proximale s'inscrit dans un secteur angulaire proximal de séparation respectif de mesure identique, de manière que lesdites encoches proximales sont régulièrement espacées les unes des autres le long dudit premier filet.

Selon une possibilité, chaque portion encochée proximale et chaque portion de séparation proximale s'inscrit dans un secteur angulaire proximal encoché ou un secteur angulaire proximal de séparation respectif de mesure identique à 5° près. Ainsi, les portions de séparation proximales et les portions encochées proximales présentent une largeur à peu près égale, et occupent respectivement une surface de taille équivalente : la stabilité primaire de la vis d'ancrage trans-pédiculaire selon l'invention est ainsi préservée.

Selon une autre possibilité, la mesure de chacun desdits secteurs angulaires proximaux encochés ou secteurs angulaires proximaux de séparation est comprise entre 30° et 75°. Dans un intervalle correspondant à un pas du premier filet, ledit premier filet présente donc environ trois encoches proximales et trois portions de séparation proximales, disposées entre lesdites encoches proximales.

D'autres modes de réalisation sont bien entendu envisageables, dans lesquels chaque encoche proximale présente une forme et/ou une taille et/ou une largeur différente, ou dans lesquels la différence de largeur entre les portions de séparation proximales et les portions encochées proximales est davantage marquée.

Dans une variante, une distance de saillie des portions de séparation proximales est entre 1 et 3 fois supérieure à une distance minimale de saillie des portions encochées proximales.

Selon une caractéristique, au moins une des encoches proximales est délimitée par un unique bord d'encoche présentant une forme d'arc de cercle concave.

Par exemple, le bord externe peut ainsi se présenter sous la forme d'une alternance de tronçons d'arcs de cercles convexes (correspondant aux portions de séparation proximales) et d'arcs de cercles concaves (correspondant aux portions encochées proximales).

On notera que, au sens de l'invention, chacune des encoches proximales ou distales présente une forme générale concave, car définie en creux dans le premier filet. Selon une autre caractéristique, au moins une des encoches proximales est délimitée par un bord d'encoche présentant une forme d'arc de cercle convexe.

Par exemple, le bord externe peut présenter au niveau de chaque portion de séparation proximale une forme d'arc de cercle convexe de rayon donné, et chaque encoche proximale peut présenter un bord d'encoche en forme d'arc de cercle convexe de rayon inférieur audit rayon, ledit bord d'encoche joignant ledit bord externe par l'intermédiaire de deux rebords latéraux s'étendant radialement par rapport à l'axe de vis.

Avantageusement, la partie proximale et la partie distale sont contiguës, l'une des portions de séparation proximales de la partie proximale étant contiguë à l'une des portions encochées distales de la partie distale.

Ainsi, la vis d'ancrage trans-pédiculaire selon l'invention ne présente pas de portion intermédiaire, dans laquelle le premier filet ne comporterait ni encoche distale ni encoche proximale : la stabilité secondaire de ladite vis d'ancrage trans-pédiculaire est donc améliorée sur toute l'étendue de cette dernière selon l'axe de vis.

Dans un mode de réalisation, chacune des portions de séparation distales s'inscrit dans un secteur angulaire distal de séparation dont la mesure est égale à 0°, de manière que les encoches distales successives sont contiguës.

Dans ce mode de réalisation, la surface occupée par les portions de séparation distales est donc minimale, favorisant par-là la stabilité secondaire de la vis d'ancrage trans-pédiculaire et augmentant l'effet du remodelage osseux en périphérie de la partie distale du premierfilet, les saillies radiales formées par les encoches distales étant alors davantage pointues et acérées.

Dans une variante, une distance de saillie des portions de séparation distales est entre 1 et 3 fois supérieure à une distance minimale de saillie des portions encochées distales. Selon une caractéristique, au moins une des encoches distales est délimitée par un unique bord d'encoche présentant une forme d'arc de cercle concave.

Dans une variante, chaque encoche distale présente une forme identique.

Par exemple, le bord externe peut se présenter (dans la partie distale du premier filet) sous la forme d'une succession ininterrompue de tronçons en forme d'arc de cercle concaves (chacun d'entre eux définissant en creux une encoche distale), chaque portion de séparation étant réduite au point de contact entre lesdits tronçons.

Selon une autre caractéristique, chaque portion encochée distale s'inscrit dans un secteur angulaire encoché dont la mesure est comprise entre 50° et 75°.

Dans un intervalle correspondant au pas du premier filet, ledit premier filet présente donc environ six encoches proximales contiguës, définissant entre elles six saillies radiales.

D'autres modes de réalisation sont bien entendu envisageables, dans lesquels chaque encoche distale présente une forme et/ou une taille et/ou une largeur variable.

Selon une possibilité, les portions encochées proximales et les portions de séparation proximales de la partie proximale présentent une épaisseur, mesurée selon l'axe de vis, supérieure à une épaisseur des portions encochées distales et des portions de séparation distales de la partie distale.

Selon une autre possibilité, le corps de vis présente une dimension transversale, mesurée perpendiculairement à l'axe de vis, dont la mesure diminue progressivement entre l'extrémité proximale et l'extrémité distale.

La partie distale du premier filet présente donc une épaisseur et un diamètre (mesuré transversalement à l'axe de vis) inférieurs à ceux de la partie proximale : de la sorte, la partie distale n'endommage pas, lors de son insertion dans le pédicule d'une vertèbre avant d'atteindre le corps spongieux du corps vertébral de cette vertèbre, la structure osseuse de ce dernier correspondant à la zone d'implantation finale de la partie proximale.

Ainsi, la stabilité primaire de la vis d'ancrage trans-pédiculaire n'est pas dégradée par le passage préalable de la partie distale du premier filet dans le pédicule d'une vertèbre. Avantageusement, la partie proximale et la partie distale du premier filet s'étendent respectivement, selon l'axe de vis, sur une longueur proximale et une longueur distale, ladite longueur proximale présentant une mesure comprise entre 0,5 et 1,5 fois celle de ladite longueur distale.

Selon une possibilité, la vis d'ancrage trans-pédiculaire comporte une tête de vis positionnée sur l'extrémité proximale du corps de vis, ladite tête de vis présentant une collerette délimitée par un bord de collerette de forme convexe et présentant des encoches de tête ménagées dans ledit bord de collerette,
ladite collerette présentant une dimension transversale, mesurée perpendiculairement à l'axe de vis, supérieure à une dimension transversale du premier filet, également mesurée perpendiculairement à l'axe de vis.

Une telle tête de vis munie d'une telle collerette permet de renforcer la stabilité primaire de la vis d'ancrage trans-pédiculaire en étant implantée dans la paroi corticale d'un pédicule d'une vertèbre, et également de favoriser une régénération osseuse à l'intérieur des encoches de tête ménagées dans ladite collerette, améliorant également la stabilité secondaire de ladite vis d'ancrage trans-pédiculaire.

Dans un mode de réalisation, l'une au moins d'une encoche proximale et d'une encoche distale est au moins partiellement comblée par un matériau biorésorbable.

Selon une possibilité, chacune des encoches proximales et chacune des encoches distales est au moins partiellement comblée par le matériau biorésorbable.

Le premierfilet présente alors, avant l'implantation de la vis d'ancrage trans-pédiculaire selon l'invention dans une vertèbre, une forme substantiellement non-encochée, chaque encoche proximale et distale étant comblée par le matériau biorésorbable : la vis d'ancrage trans-pédiculaire présente alors un profil standard substantiellement similaire à celui d'autres vis d'ancrage connues de l'état de la technique.

Dans ce mode de réalisation, la forme et/ou la disposition des encoches proximales et distales n'a donc pas d'influence lors de l'implantation de la vis d'ancrage trans-pédiculaire dans une vertèbre.

En particulier, tout risque d'endommagement de la structure osseuse du pédicule par la partie distale du premier filet est évité.

Une fois la vis d'ancrage trans-pédiculaire selon l'invention implantée dans une vertèbre et après un certain intervalle de temps, le matériau bio-résorbable est résorbé de manière à permettre une régénération osseuse de la structure osseuse de ladite vertèbre dans les encoches proximales et distales du premier filet ainsi révélées. Selon une caractéristique, le matériau biorésorbable est un polymère, par exemple un polymère de type PLA (acide polyactique), un polymère de type PLGA (acide polyglycolique) ou un copolymère de type PLA-PLGA.

Il est à noter que, dans une variante de l'invention, un tel matériau biorésorbable peut être employé avec une vis d'ancrage osseux ayant au moins un filet dans lequel sont prévues des encoches, et où ces encoches sont au moins partiellement comblées par le matériau biorésorbable.

Autrement dit, une telle variante de l'invention consisterait en une vis d'ancrage osseux, par exemple une vis d'ancrage trans-pédiculaire, adaptée pour un ancrage dans un os, comportant un corps de vis s'étendant selon un axe de vis entre une extrémité proximale et une extrémité distale, ce corps de vis présentant au moins un filet de forme hélicoïdale avec un pas de vis donné, s'étendant entre l'extrémité proximale et l'extrémité distale et présentant un bord externe de forme convexe, où ce bord externe présente une succession d'encoches, et où l'une au moins des encoches est au moins partiellement comblée par un matériau biorésorbable.

Selon une possibilité, chacune des encoches est au moins partiellement comblée par le matériau biorésorbable.

Les différentes caractéristiques évoquées précédemment pour l'invention, comme par exemple la présence d'un deuxième filet ou la présence d'une portion proximale et d'une portion distale ayant des encoches différentes, pourraient s'appliquer ensuite à cette variante de réalisation.

D'autres caractéristiques et avantages de la présente invention apparaîtront à la lecture de la description détaillée ci-après d'exemples de mise en oeuvre non limitatifs, faite en référence aux figures annexées dans lesquelles :
[Fig. 1] est une vue d'une vis d'ancrage trans-pédiculaire selon l'invention implantée dans une vertèbre ;
[Fig. 2] est une vue en perspective d'une vis d'ancrage trans-pédiculaire selon l'invention ;
[Fig. 3] est une vue de détail de la vis d'ancrage trans-pédiculaire illustrée par la figure 2 ;
[Fig. 4] est une représentation schématique du premier filet dans sa partie proximale selon un premier mode de réalisation ;
[Fig. 5] est une représentation schématique du premier filet dans sa partie proximale selon un deuxième mode de réalisation ;
[Fig. 6] est une représentation schématique du premier filet dans sa partie distale ;
[Fig. 7] est une vue de profil d'une vis d'ancrage trans-pédiculaire selon l'invention ; et
[Fig. 8] est une vue de profil d'une vis d'ancrage trans-pédiculaire selon un mode de
réalisation alternatif de l'invention.

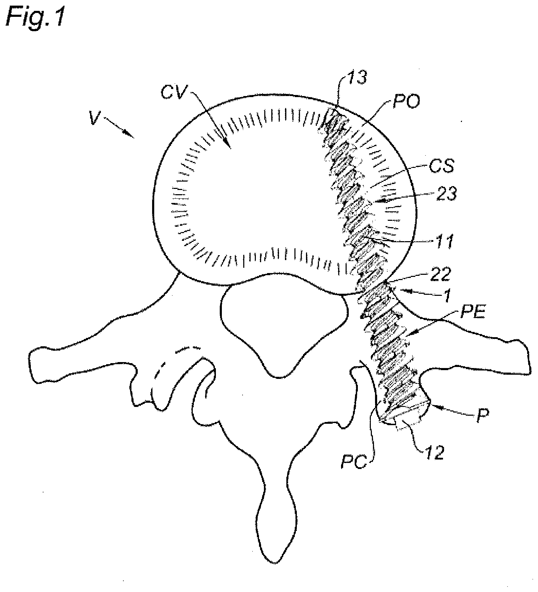

La figure 1 représente une vis d'ancrage trans-pédiculaire 1 selon l'invention implantée dans une vertèbre V d'un patient par un abord postérieur.

La vis d'ancrage trans-pédiculaire 1 présente un corps de vis 11 pénétrant un pédicule P et un corps vertébral CV de la vertèbre V, de sorte que ledit corps de vis 11 traverse quatre types de structures osseuses de la vertèbre V, chacune présentant des propriétés mécaniques différentes :
- une paroi corticale PC du pédicule P, formée d'un os compact et dur,
- une portion centrale PE du pédicule P, formée d'un os spongieux assez dense,
- un corps spongieux CS du corps vertébral CV, formé d'un os très peu dense et fragile, et
- la paroi corticale PO du corps vertébral CV, formée d'un os compact et dur.

La vis d'ancrage trans-pédiculaire 1 présente également une tête de vis 12 saillante du pédicule P et adaptée pour coopérer avec un implant chirurgical (non représenté), afin de permettre une fixation dudit implant chirurgical sur la vertèbre V.

Enfin, la vis d'ancrage trans-pédiculaire 1 présente une pointe d'attaque 13 ancrée dans la paroi corticale PO du corps vertébral CV.

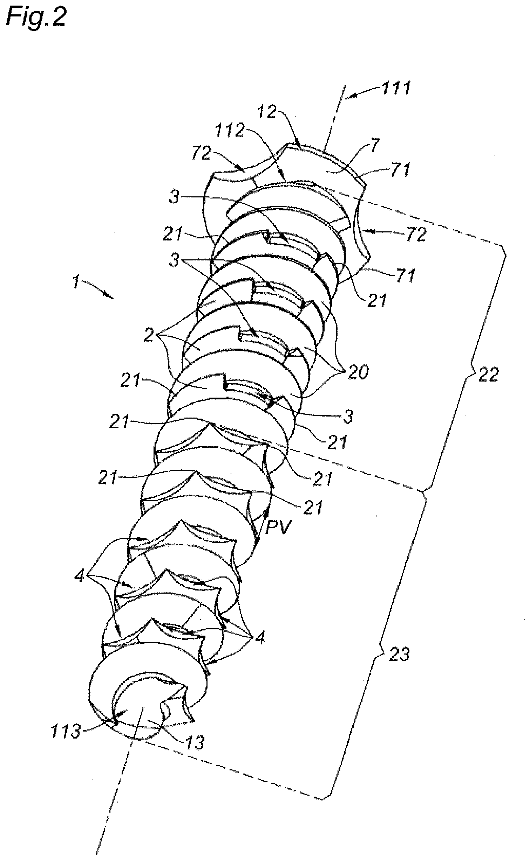

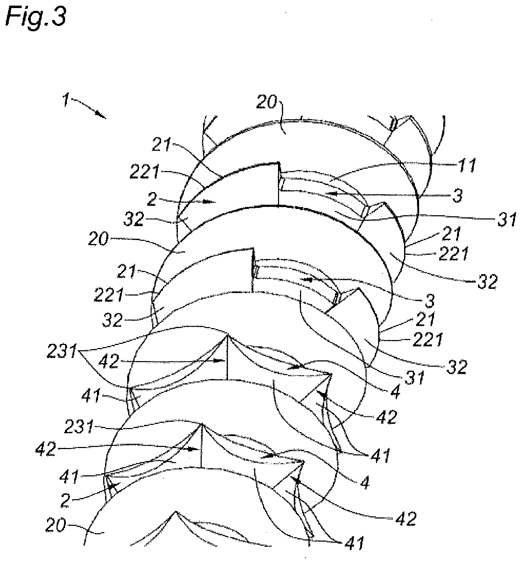

En référence aux figures 2 et 3, la vis d'ancrage trans-pédiculaire 1 présente une géométrie adaptée pour coopérer avec les différentes structures osseuses de la vertèbre V avec lesquelles elle est en contact lorsque celle-ci est implantée dans ladite vertèbre V.

Plus précisément, le corps de vis 11 de la vis d'ancrage trans-pédiculaire 1 présente une forme profilée, de section circulaire, s'étendant selon un axe de vis 111 entre une extrémité proximale 112 et une extrémité distale 113 et est muni d'un premier filet 2 et d'un deuxième filet 20, tous deux de forme hélicoïdale et disposés autour du corps de vis 111 entre la tête de vis 12 et la pointe d'attaque 13.

Il est à noter que ce corps de vis 11 peut être plein. En variante, corps de vis 11 peut être creux, autrement dit ce corps de vis 11 peut présenter un trou ou orifice interne s'étendant surtout ou partie de sa longueur depuis son extrémité proximale 112 jusqu'à son extrémité distale 113. Si ce trou ou orifice interne s'étend sur toute la longueur et traverse également la tête de vis 12, il peut alors servir par exemple au passage d'une broche ou d'un guide.

Le premier filet 2 et le deuxième filet 20 présentent un même pas de vis PV et sont par ailleurs « imbriqués » l'un avec l'autre, de sorte que le corps de vis 11 présente, le long de l'axe de vis 111, une alternance du premier filet 2 et du deuxième filet 20.

Le premierfilet 2 présente une géométrie particulière conçue pour améliorer la stabilité secondaire de la vis d'ancrage trans-pédiculaire 1 lorsque celle-ci est implantée dans la vertèbre V, comme illustré sur la figure 1 précédente.

En effet, le premier filet 2 comporte un bord externe 21 disposé, sur toute la longueur du corps de vis 11 le long de l'axe de vis 111, à une distance D constante dudit corps de vis 11 : le corps de vis 11 présentant une section (dans un plan orthogonal à l'axe de vis 111) de forme circulaire, le bord externe 21 présente une forme de spirale circulaire « s'enroulant » autour du corps de vis 11 entre la tête de vis 12 et la pointe d'attaque 13.

Comme visible sur la figure 2, le premier filet 2 présente, le long de l'axe de vis 111, deux parties 22, 23 distinctes et disposées l'une à la suite de l'autre, dans lesquelles il présente une forme spécifique :
- une partie proximale 22, en contact avec la tête de vis 12, dans laquelle des encoches proximales 3 sont ménagées dans le bord externe 21 ; et
- une partie distale 23, prolongeant la partie proximale 22 et en contact avec la pointe d'attaque 13, dans laquelle des encoches distales 4 présentant une forme différente des encoches proximales 3 sont ménagées dans le bord externe 21.

Ainsi, le bord externe 21 (généralement de forme convexe), présente dans la partie proximale 22 du premier filet 2 une succession d'encoches proximales 3 (de forme généralement concave) séparées les unes des autres par des portions non-encochées proximales 221 du bord externe 21.

Chaque encoche proximale 3 définit donc une portion encochée proximale 31 du premier filet 2, correspondant à la portion du premier filet 2 comprise entre le corps de vis 11 et ladite encoche proximale 3.

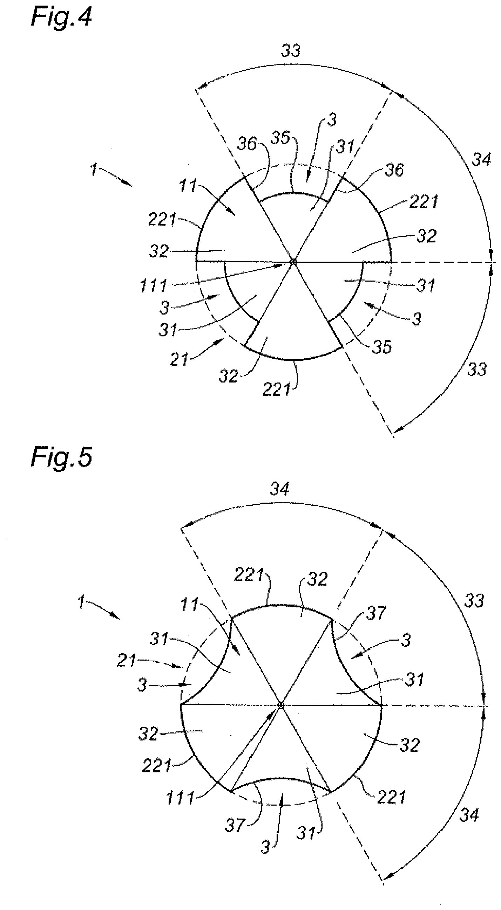

En d'autres termes, chaque portion encochée proximale 31 du premier filet 2 correspond à la portion dudit premier filet 2 comprise dans un secteur angulaire proximal encoché 33 (visible sur les figures 4 et 5) sous lequel est vue chaque encoche proximale 3 depuis l'axe de vis 111.

De même, chaque portion non-encochée proximale 221 du bord externe 21 définit une portion de séparation proximale 32 du premier filet 2, correspondant à la portion du premier filet 2 comprise entre le corps de vis 11 et ladite portion non-encochée proximale 221.

En d'autres termes, chaque portion de séparation proximale 32 du premier filet 2 correspond à la portion dudit premier filet 2 comprise dans un secteur angulaire proximal de séparation 34 (visible sur les figures 4 et 5) sous lequel est vue chaque portion non-encochée proximale 221 depuis l'axe de vis 111.

Ainsi, le premier filet 2 présente, depuis la tête de vis 12 vers la pointe d'attaque 13, une alternance de portions encochées proximales 31 et de portions de séparation proximales 32, deux portions encochées proximales 31 successives étant séparées l'une de l'autre par une portion de séparation proximale 32.

De manière similaire, le bord externe 21 (généralement de forme convexe), présente dans la partie distale 23 du premier filet 2 une succession d'encoches distales 4 (de forme généralement concave) séparées les unes des autres par des portions non-encochées distales 231 du bord externe 21.

Chaque encoche distale 4 définit donc une portion encochée distale 41 du premier filet 2, correspondant à la portion du premier filet 2 comprise entre le corps de vis 11 et ladite encoche distale 4.

En d'autres termes, chaque portion encochée distale 41 du premier filet 2 correspond à la portion dudit premier filet 2 comprise dans un secteur angulaire distal encoché 43 (visible sur les figures 4 et 5) sous lequel est vue chaque encoche distale 4 depuis l'axe de vis 111.

De même, chaque portion non-encochée distale 231 du bord externe 21 définit une portion de séparation distale 42 du premier filet 2, correspondant à la portion du premier filet 2 comprise entre le corps de vis 11 et ladite portion non-encochée distale 231.

En d'autres termes, chaque portion de séparation distale 42 du premier filet 2 correspond à la portion dudit premier filet 2 comprise dans un secteur angulaire distal de séparation 44 (visible sur les figures 4 et 5) sous lequel est vue chaque portion non-encochée distale 231 depuis l'axe de vis 111.

Ainsi, le premier filet 2 présente, dans sa partie distale 23 une alternance de portions encochées distales 41 et de portions de séparation distales 42, deux portions encochées distales 41 successives étant séparées l'une de l'autre par une portion de séparation distale 42.

La présence des encoches proximales 3 et des encoches distales 4 permet d'améliorer la stabilité secondaire de la vis d'ancrage trans-pédiculaire 1 selon l'invention.

En effet, une fois celle-ci implantée dans la vertèbre V, ces encoches proximales 3 et ces encoches distales 4 favorisent la régénération osseuse de ladite vertèbre V : quelque temps après l'opération d'implantation dans la vertèbre V, ces encoches proximales 3 et ces encoches distales 4 sont au moins partiellement comblées par de nouvelles structures osseuses.

Du fait de leur contact étendu avec le premier filet 2, ces nouvelles structures osseuses augmentent l'intensité des efforts mécaniques exercés entre la vertèbre V et la vis d'ancrage trans-pédiculaire 1, améliorant ainsi la stabilité secondaire de la vis d'ancrage trans-pédiculaire 1.

A l'inverse, les portions de séparation proximales 32 et les portions de séparation distales 42 présentent dès l'implantation de la vis d'ancrage trans-pédiculaire 1 dans la vertèbre V une surface de contact étendue avec celle-ci et participent donc de la stabilité primaire de cette vis d'ancrage trans-pédiculaire 1.

Ainsi, plus la surface occupée par les encoches proximales 3 et distales 4 est importante, plus la vis d'ancrage trans-pédiculaire présentera une stabilité secondaire importante, mais l'augmentation de la taille de ces encoches proximales 3 et distales 4 entraîne une diminution de la surface occupée par les portions de séparation proximales 32 et les portions de séparation distales 42 : l'amélioration de la stabilité secondaire de la vis d'ancrage trans-pédiculaire 1 au moyen des encoches proximales 3 et distales 4 se fait donc au détriment de la stabilité primaire de celle-ci.

La géométrie particulière du premier filet 2 dans sa partie proximale 22 et dans sa partie distale 23 répond donc à la recherche d'une optimisation de la taille de la surface occupée par les encoches proximales 3 et distales 4 par rapport à celle de la surface occupée par les portions de séparation proximales 32 et les portions de séparation distales 42, afin d'améliorer la stabilité secondaire de la vis d'ancrage trans-pédiculaire 1 sans détériorer sa stabilité primaire.

Comme visible sur la figure 1, la partie distale 23 du premier filet 2 est implantée dans le corps spongieux CS du corps vertébral CV : cette structure osseuse étant formée d'un os fragile et de faible densité, la stabilité primaire résultant du contact entre le corps spongieux CS et les portions de séparation distales 42 est donc très faible quelle que soit la taille des portions de séparation distales 42.

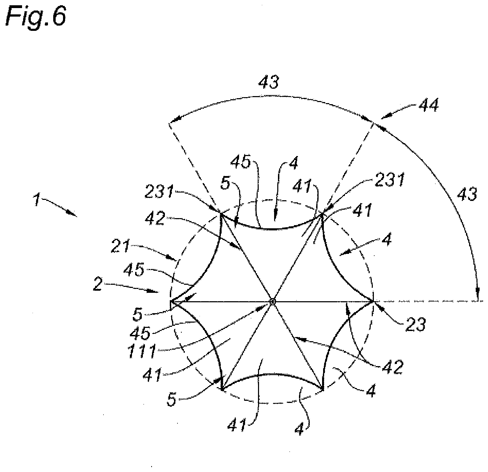

Il est ainsi possible de réduire de manière importante la surface de ces portions de séparation distales 42 sans que la stabilité primaire globale de la vis d'ancrage trans-pédiculaire 1 ne soit grandement dégradée : il est donc avantageux d'augmenter la taille des encoches distales 4 relativement à celle des portions de séparation distales 42, afin d'augmenter de manière significative la stabilité secondaire procurée par la partie distale 23 du premier filet 2.Comme visible sur la figure 6, représentant le premier filet 2 vu depuis l'axe de vis 111 sur une longueur d'un pas de vis PV, les encoches distales 4 successives sont en fait contiguës : chaque portion non-encochée distale 231 du bord externe 21 est ainsi réduite, dans la partie distale 23, à un unique point de contact entre deux encoches distales 4 successives.

Chaque portion de séparation distale 42 est ainsi également réduite à un segment de droite joignant ce point de contact et le corps de vis 11 : la surface occupée les portions de séparation distales 42 est donc nulle
De même, la mesure des secteurs angulaires distaux de séparation 44, dans lesquels sont inscrites les portions de séparation distales 42, est nulle.

A l'inverse, les encoches distales 4 sont présentes en grand nombre et présentent une taille importante : les secteurs angulaires distaux encochés 43, sous lesquels sont vus les encoches distales 4 et dans lesquels sont inscrites les portions encochées distales 41, présentent une mesure égale à 60°.

Sur une longueur du premier filet 2 correspondant à un pas de vis PV, le premier filet 2 présente ainsi six encoches distales contiguës (et également six portions encochées distales 41). On notera que, dans le mode de réalisation représenté sur la figure 6, les encoches distales 4 présentent toutes la même forme et la même taille, et les secteurs angulaires distaux encochés 43 ont la même mesure : d'autres modes de réalisation sont évidemment envisageables.

Ainsi, dans la partie distale 23, sur cette longueur du premier filet 2 correspondant à un pas de vis PV, un rapport entre la somme des mesures de tous les secteurs angulaires distaux de séparation 44 et la somme des mesures de tous les secteurs angulaires distaux encochés 43 est égal à 0 (et est donc en particulier compris entre 0% et 10%) : dans la partie distale 23 du premier filet 2, les portions encochées distales 41 occupent donc une grande surface relativement aux portions de séparation distales 42, permettant de fortement augmenter la stabilité secondaire procurée par cette partie distale 23.

A l'inverse, comme visible sur la figure 1, la partie proximale 22 du premier filet 2 est implantée dans le pédicule P de la vertèbre V et est donc en contact avec la paroi corticale PC et la portion centrale PE : ces structures osseuses étant formées d'un os dense et solide, la stabilité primaire résultant du contact entre le pédicule P et les portions de séparation proximales 32 est donc très importante et proportionnelle à la taille de la surface occupée par celles-ci.

Il n'est alors pas possible de réduire de manière importante la surface de ces portions de séparation proximales 32 sans que la stabilité primaire globale de la vis d'ancrage trans-pédiculaire 1 ne soit grandement dégradée : il est donc nécessaire de limiter la taille des encoches proximales 3 et de conserver une taille importante des portions de séparation proximales 32, afin d'augmenter légèrement la stabilité secondaire procurée par la partie proximale 22 du premier filet 2 tout en conservant une bonne stabilité primaire.

Comme visible sur les figures 4 et 5, représentant respectivement le premier filet 2 vu depuis l'axe de vis 111 sur une longueur d'un pas de vis PV selon un premier mode de réalisation et selon un deuxième mode de réalisation, les encoches proximales 3 successives ne sont pas contiguës et sont séparées par des portions non encochées 221 du bord externe 21 de longueur non nulle : les portions encochées proximales 31 successives sont ainsi séparées par une portion de séparation proximale 32 de taille importante.

Dans les deux modes de réalisations représentés sur les figures 4 et 5, les secteurs angulaires proximaux de séparation 34, dans lesquels sont inscrites les portions de séparation proximales 32, et les secteurs angulaires proximaux encochés 33, dans lesquels sont inscrites les portions encochées proximales 31, présentent une même mesure, égale à 60°.

Ainsi, dans la partie proximale 22, sur cette longueur du premier filet 2 correspondant à un pas de vis PV, un rapport entre la somme des mesures de tous les secteurs angulaires proximaux de séparation 34 et la somme des mesures de tous les secteurs angulaires proximaux encochés 33 est égal à 1 (et est donc en particulier compris entre 0% et 150%).

Sur une longueur du premier filet 2 correspondant à un pas de vis PV, le premier filet 2 présente ainsi seulement trois encoches proximales 3 (et donc trois portions encochées proximales 31).

On notera que, dans le mode de réalisation représenté sur les figure 4 et 5, les encoches proximales 3 présentent toutes la même forme et la même taille, et les secteurs angulaires proximaux encochés 33 ont la même mesure : d'autres modes de réalisation sont évidemment envisageables.

De même, les portions de séparation proximales 32 présentent toutes la même forme et la même taille, et les secteurs angulaires proximaux de séparation 34 ont la même mesure : d'autres modes de réalisation sont également envisageables.

L'équilibre entre la surface occupée par les portions de séparation proximales 32 et celle occupée par les portions encochées proximales 31 permet ainsi d'améliorer la stabilité secondaire procurée par la partie proximale 22 de la vis d'ancrage trans-pédiculaire 1, tout en conservant une bonne stabilité primaire.

On notera que sur les figures 4, 5 et 6, le bord externe 21 est représenté, dans les portions encochées proximales 31 et les portions encochées distales 41, par un trait en pointillés (coïncidant avec les portions non encochées 221 et les portions non encochées 231 respectivement dans les portions de séparation proximales 31 et les portions de séparation distales 41) : c'est par rapport à ce bord externe 21 que sont définies les encoches proximales 3 et distales 4.

De nombreux modes de réalisation sont envisageables concernant la forme particulière de ces encoches proximales 3 et distales 4.

Par exemple, dans le premier mode de réalisation représenté par la figure 4, chaque encoche proximale 3 est délimitée par :
- un bord d'encoche 35 en forme d'arc de cercle convexe, et
- deux rebords latéraux 36 s'étendant radialement par rapport à l'axe de vis 111 et joignant le bord d'encoche 35 et les portions non-encochées 221 adjacentes à l'encoche proximale 3.

Dans le deuxième mode de réalisation représenté par la figure 5, chaque encoche proximale 3 est délimitée par un unique un bord d'encoche 37 en forme d'arc de cercle concave, joignant les portions non-encochées 221 adjacentes à l'encoche proximale 3. De manière similaire, comme visible sur la figure 6, chaque encoche distale 4 est délimitée par un unique un bord d'encoche 45 en forme d'arc de cercle concave, joignant les portions non-encochées 231 adjacentes à l'encoche proximale 4.

On notera que, du fait que les encoches distales 4 sont contiguës, le premier filet 2 présente dans la partie distale 23 une forme « en étoile », deux encoches distales 4 successives formant entre elles une saillie radiale 5 en forme de pointe.

Comme précédemment évoqué, ces saillies radiales 5 permettent de créer à leur périphérie une zone de remodelage osseux favorisant également la stabilité secondaire de la vis d'ancrage trans-pédiculaire 1.

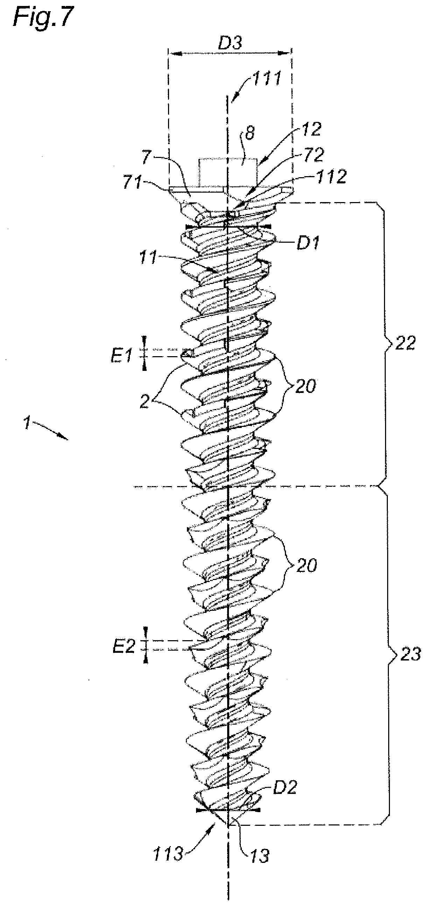

Par ailleurs, comme visible sur la figure 7, le premier filet 2 présente dans sa partie proximale 22 une épaisseur E1, mesurée selon l'axe de vis 111, supérieur à une épaisseur E2 du même premier filet 2 dans sa partie distale 23.

De plus, le corps de vis 11 présente une forme légèrement conique et présente une dimension transversale (correspondant au diamètre de sa section circulaire), mesurée perpendiculairement à l'axe de vis 111, dont la mesure diminue progressivement entre l'extrémité proximale 112 et l'extrémité distale 113.

Le corps de vis 11 présente ainsi une dimension transversale maximale D1 à l'extrémité proximale 112 et une dimension transversale minimale D2 à l'extrémité distale 113.

Cette réduction de la dimension transversale du corps de vis et de l'épaisseur du premier filet 2 entre la partie proximale 22 et la partie distale 23 permet d'éviter que la partie distale 23 n'endommage la structure osseuse du pédicule P (dans lequel la partie proximale 22 sera finalement implantée) pendant l'introduction de la vis d'ancrage trans-pédiculaire 1 dans la vertèbre V.

De la sorte, la stabilité primaire de la vis d'ancrage trans-pédiculaire 1 procurée par la partie proximale 22 n'est pas détériorée par le passage antérieur de la partie distale 23 dans le pédicule P.

La tête de vis 12 présente également une structure adaptée pour favoriser la stabilité primaire et secondaire de la vis d'ancrage trans-pédiculaire 1.

En effet, cette tête de vis présente notamment une collerette 7 délimitée par un bord de collerette 71, dans lequel sont ménagées des encoches de tête 72.

Cette collerette 7 présente une dimension transversale D3 de mesure supérieure à la dimension transverse maximale D1 du premier filet 2 : cette collerette 7, une fois implantée dans la paroi corticale PC du pédicule P, présente ainsi une grande surface de contact avec la structure osseuse de ce pédicule P et contribue de manière importante à la stabilité primaire de la vis d'ancrage trans-pédiculaire 1.

De la même manière que les encoches proximales 3 ou distales 4 du premier filet 2, les encoches de tête 72 favorisent une régénération osseuse du pédicule P après l'implantation de la vis d'ancrage trans-pédiculaire 1 dans celui-ci, contribuant ainsi à la stabilité secondaire de cette vis d'ancrage trans-pédiculaire 1.

On notera que la géométrie de la tête 12 peut également être illustrée par la figure 5, les encoches proximales 3 représentées (ménagées dans le bord externe 21) correspondant alors aux encoches de tête 72 ménagées dans le bord de collerette 71. Bien évidemment, de nombreux autres modes de réalisation concernant la géométrie de cette collerette 7 sont possibles.

On notera que la tête de vis 12 présente également un embout 8, destiné à saillir du pédicule P une fois la vis d'ancrage trans-pédiculaire 1 implantée dans la vertèbre V, ledit embout 8 étant adapté pour coopérer avec un implant chirurgical destiné à être fixé sur ladite vertèbre V au moyen de la vis d'ancrage trans-pédiculaire 1.

La stabilité primaire de la vis d'ancrage trans-pédiculaire 1 est également assurée par la présence du deuxième filet 20.

En effet, comme visible sur les figures 2, 3 et 7, ce deuxième filet 20 présente une forme similaire à celle du premier filet 2, mais est dépourvu d'encoches proximales ou distales. Ce deuxième filet 20 présente donc une surface de contact très étendue avec les structures osseuses de la vertèbre V dans laquelle la vis d'ancrage trans-pédiculaire 1 est implantée, garantissant une très bonne stabilité primaire à cette vis d'ancrage trans-pédiculaire 1.

En revanche, ce deuxième filet 20 ne contribue que marginalement à la stabilité secondaire de la vis d'ancrage trans-pédiculaire 1.

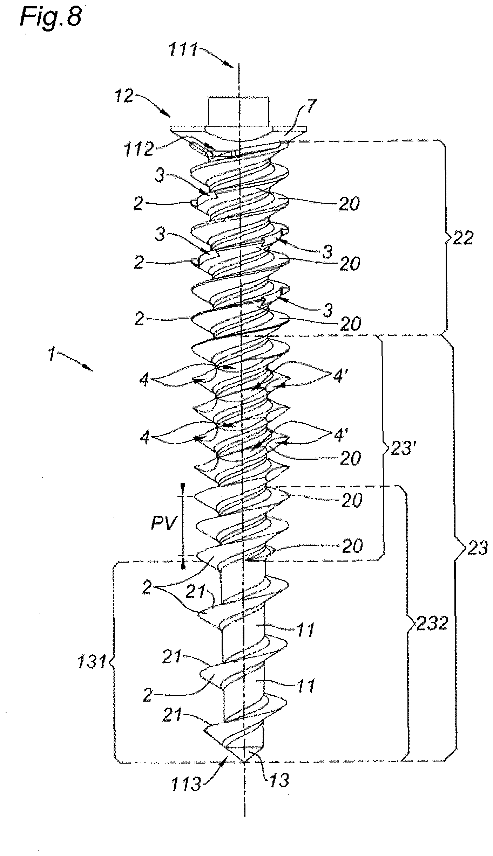

La figure 8 représente un mode de réalisation alternatif de l'invention, identique au mode de réalisation précédemment décrit par les figures 1 à 6 à l'exception de la géométrie et de la structure du premier filet 2 et du deuxième filet 20.

En particulier, le premier filet 2 présente ici, dans sa partie distale 23, une sous-partie distale 232 à proximité de la pointe d'attaque 13 dans laquelle le bord externe 21 ne comporte aucune encoche distale 4.

Le premier filet 2 présente cependant des encoches distales 4, identiques à celles précédemment décrites et visibles notamment sur les figures 2, 3 ou 6 et ayant la même fonction, dans le reste de sa partie distale 23, à proximité de sa partie proximale 22. Par ailleurs, dans ce mode de réalisation alternatif, le deuxième filet 20 ne s'étend pas jusqu'à la pointe d'attaque 13 et présente ainsi une partie distale 23' plus courte que la partie distale 23 du premier filet 2.

La vis d'ancrage trans-pédiculaire 1 de la figure 8 présente ainsi une portion de pointe 131 ne présentant qu'un unique filet dépourvu d'encoches, à savoir une portion de la sous-partie distale 232 du premier filet 2 : cette caractéristique permet de favoriser et de faciliter la pénétration de ladite vis d'ancrage trans-pédiculaire 1 dans la structure osseuse d'une vertèbre V.

De plus, du fait de l'absence d'encoches distales 4 dans cette portion de pointe 131, la vis d'ancrage trans-pédiculaire 1 ne présente aucune saillie radiale en forme de pointe à proximité de la pointe d'attaque 13, ce qui permet de limiter le risque de détérioration des structures osseuses que ladite vis d'ancrage trans-pédiculaire 1 est amenée à traverser lors de son implantation dans une vertèbre V.

Enfin, le deuxième filet 20 présente, dans sa partie distale 23', des encoches distales 4' ayant la même forme et la même fonction que les encoches distales 4 ménagées dans le premier filet 2 et décrites précédemment.

En particulier, la géométrie et la répartition de ces encoches distales 4' peuvent être illustrées par celles des encoches distales 4 représentées sur la figure 6.

Ce deuxième filet 20 présente donc également, dans sa partie distale 23', une forme « en étoile » permettant de maximiser la stabilité secondaire procurée à la vis d'ancrage trans-pédiculaire 1.

Ainsi, la stabilité secondaire globale de la vis d'ancrage trans-pédiculaire 1 de la figure 8 est équivalente à celle du mode de réalisation précédent car bien que cette vis d'ancrage trans-pédiculaire 1 ne comporte aucune encoche distale à proximité de la pointe d'attaque 13, elle en comporte un nombre bien plus important à proximité de la partie proximale 22.

Par ailleurs, et de la même manière que dans le mode de réalisation précédent, le deuxième filet 20 ne présente aucune encoche proximale dans sa partie distale 22 (tandis que le premier filet 2 présente des encoches proximales 3 identiques à celles précédemment décrites et visibles notamment sur les figures 2 à 5) : de ce fait, la partie proximale 22 du deuxième filet 20 ne participe que très faiblement à la stabilité secondaire de la vis d'ancrage trans-pédiculaire 1, mais permet de garantir une forte stabilité primaire grâce aux larges surfaces de contact qu'elle présente avec les structures osseuses environnantes une fois cette vis d'ancrage trans-pédiculaire 1 implantée dans une vertèbre V.

Ainsi, ce mode de réalisation alternatif permet de faciliter l'insertion de la vis d'ancrage trans-pédiculaire 1 dans une vertèbre V en évitant de détériorer cette dernière, tout en présentant une stabilité primaire et une stabilité secondaire sensiblement équivalentes au mode de réalisation précédent.

Comme précédemment décrit, il est par ailleurs envisageable que, dans l'un ou l'autre des modes de réalisation précédemment décrits, les encoches proximales 3 et les encoches distales 4 ou 4' soient au moins partiellement comblées, avant implantation de la vis d'ancrage trans-pédiculaire 1 dans la vertèbre V, par un matériau biorésorbable, par exemple un polymère de type PLA (acide polyactique), un polymère de type PLGA (acide polyglycolique) ou un copolymère de type PLA-PLGA. De préférence, les encoches proximales 3 et les encoches distales 4 ou 4' sont intégralement comblées de sorte que le matériau biorésorbable procure une continuité avec le bord externe 21 du premier filet 2, et éventuellement avec le bord externe du deuxième filet 20 dans le cas où ce dernier est également muni d'encoches.

De la sorte, pendant l'introduction de la vis d'ancrage trans-pédiculaire 1 dans la vertèbre V, tout risque d'endommagement de la structure osseuse de celle-ci du fait de la présence des encoches proximales 3 ou des encoches distales 4 ou 4' est évité.

Après l'opération implantation de la vis d'ancrage trans-pédiculaire 1 dans la vertèbre V, le matériau biorésorbable se résorbe et les encoches proximales 3 et distales 4 ou 4' se découvrent et peuvent devenir le lieu d'une régénération osseuse contribuant, comme cela est décrit plus haut, à la stabilité secondaire de la vis d'ancrage trans-pédiculaire 1.

Ainsi, la vis d'ancrage trans-pédiculaire 1 selon l'invention présente une géométrie adaptée aux différentes structures osseuses de la vertèbre V dans laquelle elle est destinée à être implantée, ladite géométrie permettant de grandement améliorer la stabilité secondaire de cette vis d'ancrage trans-pédiculaire 1 par rapport aux vis d'ancrage de l'état de la technique, sans dégrader sa stabilité primaire.

## Revendications

1. Vis d'ancrage trans-pédiculaire (1), adaptée pour un ancrage dans un pédicule (P) d'une vertèbre (V), comportant un corps de vis (11) s'étendant selon un axe de vis (111) entre une extrémité proximale (112) et une extrémité distale (113), ledit corps de vis (11) présentant un premier filet (2) et un deuxième filet (20), chacun desdits premier filet (2) et deuxième filet (20) étant de forme hélicoïdale avec un même pas de vis (PV), s'étendant entre ladite extrémité proximale (112) et ladite extrémité distale (113) et présentant un bord externe (21) de forme convexe,
ladite vis d'ancrage trans-pédiculaire (1) étant **caractérisée en ce qu'**au moins ledit premier filet (2) présente, depuis l'extrémité proximale (112) vers l'extrémité distale (113), au moins :
- une partie proximale (22), dans laquelle le bord externe (21) dudit premier filet (2) présente une succession de portions encochées proximales (31), chacune définie par une encoche proximale (3) ménagée dans ledit bord externe (21) et s'inscrivant dans un secteur angulaire proximal encoché (33), où deux portions encochées proximales (31) successives sont séparées l'une de l'autre par une portion de séparation proximale (32), dans laquelle ledit bord externe (21) n'est pas encoché et s'inscrivant dans un secteur angulaire proximal de séparation (34),
et dans laquelle, sur une longueur du premier filet (2) correspondant à un pas de vis (PV), un rapport entre la somme des mesures de tous les secteurs angulaires proximaux de séparation (34) et la somme des mesures de tous les secteurs angulaires proximaux encochés (33) est compris entre 50% et 150% ; et
- une partie distale (23), dans laquelle le bord externe (21) dudit premier filet (2) présente une succession de portions encochées distales (41), chacune définie par une encoche distale (4) ménagée dans ledit bord externe (21) et s'inscrivant dans un secteur angulaire distal encoché (43), où deux portions encochées distales (41) successives sont séparées par une portion de séparation distale (42), dans laquelle ledit bord externe (21) n'est pas encoché et s'inscrivant dans un secteur angulaire distal de séparation (34),
et dans laquelle, sur une longueur du premier filet (2) correspondant à un pas de vis (PV), un rapport entre la somme des mesures de tous les secteurs angulaires distaux de séparation (44) et la somme des mesures de tous les secteurs angulaires distaux encochés (43) est compris entre 0% et 10%.

2. Vis d'ancrage trans-pédiculaire (1) selon la revendication 1, dans laquelle chaque encoche proximale (3) présente une forme identique et dans laquelle chaque portion de séparation proximale (32) s'inscrit dans un secteur angulaire proximal de séparation (34) respectif de mesure identique, de manière que lesdites encoches proximales (3) sont régulièrement espacées les unes des autres le long dudit premier filet (2).

3. Vis d'ancrage trans-pédiculaire (1) selon la revendication 2, dans laquelle chaque portion encochée proximale (31) et chaque portion de séparation proximale (32) s'inscrit dans un secteur angulaire proximal encoché (33) ou un secteur angulaire proximal de séparation (34) respectif de mesure identique à 5° près.

4. Vis d'ancrage trans-pédiculaire (1) selon la revendication 3, dans laquelle la mesure de chacun desdits secteurs angulaires proximaux encochés (33) ou secteurs angulaires proximaux de séparation (34) est comprise entre 30° et 75°.

5. Vis d'ancrage trans-pédiculaire (1) selon l'une quelconque des revendications 1 à 4, dans laquelle au moins une des encoches proximales (3) est délimitée par un unique bord d'encoche (37) présentant une forme d'arc de cercle concave.

6. Vis d'ancrage trans-pédiculaire (1) selon l'une quelconque des revendications 1 à 5, dans laquelle au moins une des encoches proximales (3) est délimitée par un bord d'encoche (35) présentant une forme d'arc de cercle convexe.

7. Vis d'ancrage trans-pédiculaire (1) selon l'une quelconque des revendications 1 à 6, dans laquelle la partie proximale (22) et la partie distale (23) sont contiguës, l'une des portions de séparation proximales (32) de la partie proximale (22) étant contiguë à l'une des portions encochées distales (41) de la partie distale (23).

8. Vis d'ancrage trans-pédiculaire (1) selon l'une quelconque des revendications 1 à 7, dans laquelle chacune des portions de séparation distales (42) s'inscrit dans un secteur angulaire distal de séparation (44) dont la mesure est égale à 0°, de manière que les encoches distales (4) successives sont contiguës.

9. Vis d'ancrage trans-pédiculaire (1) selon l'une quelconque des revendications 1 à 8, dans laquelle au moins une des encoches distales (4) est délimitée par un unique bord d'encoche (45) présentant une forme d'arc de cercle concave.

10. Vis d'ancrage trans-pédiculaire (1) selon l'une quelconque des revendications 1 à 9, dans laquelle chaque portion encochée distale (41) s'inscrit dans un secteur angulaire distal encoché (43) dont la mesure est comprise entre 50° et 75°.

11. Vis d'ancrage trans-pédiculaire (1) selon l'une quelconque des revendications 1 à 10, dans laquelle les portions encochées proximales (31) et les portions de séparation proximales (32) de la partie proximale (22) présentent une épaisseur (E1), mesurée selon l'axe de vis (111), supérieure à une épaisseur (E2) des portions encochées distales (41) et des portions de séparation distales (42) de la partie distale (23).

12. Vis d'ancrage trans-pédiculaire (1) selon l'une quelconque des revendications 1 à 11, dans laquelle la partie proximale (22) et la partie distale (23) du premier filet (2) s'étendent respectivement, selon l'axe de vis (111), sur une longueur proximale et une longueur distale, ladite longueur proximale présentant une mesure comprise entre 0,5 et 1,5 fois celle de ladite longueur distale.

13. Vis d'ancrage trans-pédiculaire (1) selon l'une quelconque des revendications 1 à 12, dans laquelle le corps de vis (11) présente une dimension transversale (D1, D2), mesurée perpendiculairement à l'axe de vis (111), dont la mesure diminue progressivement entre l'extrémité proximale (112) et l'extrémité distale (113).

14. Vis d'ancrage trans-pédiculaire (1) selon l'une quelconque des revendications 1 à 13, comportant une tête de vis (12) positionnée sur l'extrémité proximale (112) du corps de vis (11), ladite tête de vis (12) présentant une collerette (7) délimitée par un bord de collerette (71) de forme convexe et présentant des encoches de tête (72) ménagées dans ledit bord de collerette (71),
ladite collerette (7) présentant une dimension transversale (D3), mesurée perpendiculairement à l'axe de vis (111), supérieure à une dimension transversale (D1, D2) du premier filet (2), également mesurée perpendiculairement à l'axe de vis (111).

15. Vis d'ancrage trans-pédiculaire (1) selon l'une quelconque des revendications 1 à 14, dans laquelle l'une au moins d'une encoche proximale (3) et d'une encoche distale (4) est au moins partiellement comblée par un matériau biorésorbable.

16. Vis d'ancrage trans-pédiculaire (1) selon la revendication 15, dans laquelle chacune des encoches proximales (3) et chacune des encoches distales (4) est au moins partiellement comblée par le matériau biorésorbable.

17. Vis d'ancrage trans-pédiculaire (1) selon l'une quelconque des revendications 15 et 16, dans laquelle le matériau biorésorbable est un polymère, par exemple un polymère de type PLA (acide polyactique), un polymère de type PLGA (acide polyglycolique) ou un copolymère de type PLA-PLGA.

## Patentansprüche

1. Transpedikuläre Verankerungsschraube (1), die zur Verankerung in einem Pedikel (P) eines Wirbels (V) ausgelegt ist und einen Schraubenkörper (11) umfasst, der sich entlang einer Schraubenachse (111) zwischen einem proximalen Ende (112) und einem distalen Ende (113) erstreckt, wobei der Schraubenkörper (11) ein erstes Gewinde (2) und ein zweites Gewinde (20) aufweist, wobei das erste Gewinde (2) und das zweite Gewinde (20) jeweils spiralförmig mit der gleichen Gewindesteigung (PV) sind, sich zwischen dem proximalen Ende (112) und dem distalen Ende (113) erstrecken und eine konvex geformte Außenkante (21) aufweisen,
wobei die transpedikuläre Verankerungsschraube (1) **dadurch gekennzeichnet ist, dass** mindestens das erste Gewinde (2) vom proximalen Ende (112) zum distalen Ende (113) mindestens Folgendes aufweist:
- einen proximalen Teil (22), in dem die Außenkante (21) des ersten Gewindes (2) eine Reihe eingekerbter proximaler Abschnitte (31) aufweist, die jeweils durch eine proximale Einkerbung (3) definiert sind, die in der Außenkante (21) vorgesehen ist und in einen eingekerbten proximalen Winkelsektor (33) eingefügt ist, wobei zwei aufeinanderfolgende eingekerbte proximale Abschnitte (31) durch einen proximalen Trennungsabschnitt (32) getrennt voneinander sind, in dem die Außenkante (21) nicht eingekerbt ist und in einen proximalen Trennungswinkelsektor (34) eingefügt ist,
und in dem ein Verhältnis zwischen der Summe der Messungen aller proximalen Trennwinkelsektoren (34) und der Summe der Messungen aller eingekerbten proximalen Winkelsektoren (33) über eine Länge des ersten Gewindes (2), die einer Gewindesteigung (PV) entspricht, zwischen 50 % und 150 % beträgt; und
- einen distalen Teil (23), in dem die Außenkante (21) des ersten Gewindes (2) eine Reihe eingekerbter distaler Abschnitte (41) aufweist, die jeweils durch eine distale Einkerbung (4) definiert sind, die in der Außenkante (21) vorgesehen ist und in einen eingekerbten distalen Winkelsektor (43) eingefügt ist, wobei zwei aufeinanderfolgende eingekerbte distale Abschnitte (41) durch einen distalen Trennungsabschnitt (42) getrennt voneinander sind, in dem die Außenkante (21) nicht eingekerbt ist und in einen distalen Trennungswinkelsektor (34) eingefügt ist,
und in dem ein Verhältnis zwischen der Summe der Messungen aller distalen Trennwinkelsektoren (44) und der Summe der Messungen aller eingekerbten distalen Winkelsektoren (43) über eine Länge des ersten Gewindes (2), die einer Gewindesteigung (PV) entspricht, zwischen 0 % und 10 % beträgt.

2. Transpedikuläre Verankerungsschraube (1) nach Anspruch 1, wobei jede proximale Einkerbung (3) eine identische Form aufweist und wobei jeder proximale Trennungsabschnitt (32) in einen jeweiligen proximalen Trennungswinkelsektor (34) mit identischer Abmessung eingefügt ist, so dass die proximalen Einkerbungen (3) entlang des ersten Gewindes (2) gleichmäßig voneinander beabstandet sind.

3. Transpedikuläre Verankerungsschraube (1) nach Anspruch 2, wobei jeder eingekerbte proximale Abschnitt (31) und jeder proximale Trennungsabschnitt (32) in einen eingekerbten proximalen Winkelsektor (33) oder einen proximalen Trennungswinkelsektor (34) mit jeweiliger identischer Abmessung innerhalb von 5° eingefügt ist.

4. Transpedikuläre Verankerungsschraube (1) nach Anspruch 3, wobei die Abmessung jedes der eingekerbten proximalen Winkelsektoren (33) oder proximalen Trennungswinkelsektoren (34) zwischen 30° und 75° beträgt.

5. Transpedikuläre Verankerungsschraube (1) nach einem der Ansprüche 1 bis 4, wobei mindestens eine der proximalen Einkerbungen (3) durch einen einzelnen Kerbenrand (37) begrenzt ist, der eine konkave Kreisbogenform aufweist.

6. Transpedikuläre Verankerungsschraube (1) nach einem der Ansprüche 1 bis 5, wobei mindestens eine der proximalen Einkerbungen (3) durch einen Kerbenrand (35) begrenzt ist, der eine konvexe Kreisbogenform aufweist.

7. Transpedikuläre Verankerungsschraube (1) nach einem der Ansprüche 1 bis 6, wobei der proximale Teil (22) und der distale Teil (23) aneinandergrenzen, wobei einer der proximalen Trennungsabschnitte (32) des proximalen Teils (22) an einen der eingekerbten distalen Abschnitte (41) des distalen Teils (23) angrenzt.

8. Transpedikuläre Verankerungsschraube (1) nach einem der Ansprüche 1 bis 7, wobei jeder der distalen Trennungsabschnitte (42) in einen distalen Trennungswinkelsektor (44) eingefügt ist, dessen Abmessung gleich 0° ist, so dass die aufeinanderfolgenden distalen Einkerbungen (4) aneinandergrenzen.

9. Transpedikuläre Verankerungsschraube (1) nach einem der Ansprüche 1 bis 8, wobei mindestens eine der distalen Einkerbungen (4) durch einen einzelnen Kerbenrand (45) begrenzt ist, der eine konkave Kreisbogenform aufweist.

10. Transpedikuläre Verankerungsschraube (1) nach einem der Ansprüche 1 bis 9, wobei jeder eingekerbte distale Abschnitt (41) in einen eingekerbten distalen Winkelsektor (43) eingefügt ist, dessen Abmessung zwischen 50° und 75° beträgt.

11. Transpedikuläre Verankerungsschraube (1) nach einem der Ansprüche 1 bis 10, wobei die eingekerbten proximalen Abschnitte (31) und die proximalen Trennungsabschnitte (32) des proximalen Teils (22) eine Dicke (E1) aufweisen, die entlang der Schraubenachse (111) gemessen wird und größer ist als eine Dicke (E2) der eingekerbten distalen Abschnitte (41) und der distalen Trennungsabschnitte (42) des distalen Teils (23).

12. Transpedikuläre Verankerungsschraube (1) nach einem der Ansprüche 1 bis 11, wobei sich der proximale Teil (22) und der distale Teil (23) des ersten Gewindes (2) jeweils entlang der Schraubenachse (111) über eine proximale Länge und eine distale Länge erstrecken, wobei die proximale Länge eine Abmessung zwischen dem 0,5- und 1,5-Fachen der distalen Länge aufweist.

13. Transpedikuläre Verankerungsschraube (1) nach einem der Ansprüche 1 bis 12, wobei der Schraubenkörper (11) eine senkrecht zur Schraubenachse (111) gemessene Querabmessung (D1, D2) aufweist, deren Abmessung zwischen dem proximalen Ende (112) und dem distalen Ende (113) zunehmend abnimmt.

14. Transpedikuläre Verankerungsschraube (1) nach einem der Ansprüche 1 bis 13, umfassend einen Schraubenkopf (12), der am proximalen Ende (112) des Schraubenkörpers (11) positioniert ist, wobei der Schraubenkopf (12) einen Kragen (7) aufweist, der von einem konvex geformten Kragenrand (71) begrenzt wird, und Kopfeinkerbungen (72) aufweist, die in dem Kragenrand (71) vorgesehen sind,
wobei der Kragen (7) eine senkrecht zur Schraubenachse (111) gemessene Querabmessung (D3) aufweist, die größer ist als eine Querabmessung (D1, D2) des ersten Gewindes (2), die ebenfalls senkrecht zur Schraubenachse (111) gemessen wird.

15. Transpedikuläre Verankerungsschraube (1) nach einem der Ansprüche 1 bis 14, wobei mindestens eine der proximalen Einkerbung (3) und der distalen Einkerbung (4) mindestens teilweise mit einem bioresorbierbaren Material gefüllt ist.

16. Transpedikuläre Verankerungsschraube (1) nach Anspruch 15, wobei jede der proximalen Einkerbungen (3) und jede der distalen Einkerbungen (4) mindestens teilweise mit dem bioresorbierbaren Material gefüllt ist.

17. Transpedikuläre Verankerungsschraube (1) nach einem der Ansprüche 15 und 16, wobei das bioresorbierbare Material ein Polymer ist, beispielsweise ein Polymer vom Typ PLA (Polymilchsäure), ein Polymer vom Typ PLGA (Polyglykolsäure) oder ein Copolymer vom Typ PLA-PLGA.

## Claims

1. A transpedicular anchoring screw (1), adapted for anchorage in a pedicle (P) of a vertebra (V), including a screw body (11) extending along a screw axis (111) between a proximal end (112) and a distal end (113), said screw body (11) having a first thread (2) and a second thread (20), each of said first thread (2) and second thread (20) being helical-shaped with a same screw pitch (PV), extending between said proximal end (112) and said distal end (113) and having a convex-shaped outer edge (21),
said transpedicular anchoring screw (1) being **characterized in that** at least said first thread (2) has, from the proximal end (112) towards the distal end (113), at least:
- a proximal portion (22), in which the outer edge (21) of said first thread (2) has a series of notched proximal portions (31), each defined by a proximal notch (3) formed in said outer edge (21) and inscribed within a notched proximal angular sector (33), wherein two successive notched proximal portions (31) are separated from each other by a separation proximal portion (32), in which said outer edge (21) is not notched and is inscribed within a separation proximal angular sector (34),
and wherein, over a length of the first thread (2) corresponding to one screw pitch (PV), a ratio between the sum of the measurements of all of the separation proximal angular sectors (34) and the sum of the measurements of all of the notched proximal angular sectors (33) is comprised between 50% and 150%; and
- a distal portion (23), in which the outer edge (21) of said first thread (2) has a series of notched distal portions (41), each defined by a distal notch (4) formed in said outer edge (21) and inscribed within a notched distal angular sector (43), wherein two successive notched distal portions (41) are separated by a separation distal portion (42), in which said outer edge (21) is not notched and is inscribed within a separation distal angular sector (34),
and wherein, over a length of the first thread (2) corresponding to one screw pitch (PV), a ratio between the sum of the measurements of all of the separation distal angular sectors (44) and the sum of the measurements of all of the notched distal angular sectors (43) is comprised between 0% and 10%.

2. The transpedicular anchoring screw (1) according to claim 1, wherein each proximal notch (3) has an identical shape and wherein each separation proximal portion (32) is inscribed within a respective separation proximal angular sector (34) with an identical extent, so that said proximal notches (3) are evenly spaced from each other along said first thread (2).

3. The transpedicular anchoring screw (1) according to claim 2, wherein each notched proximal portion (31) and each separation proximal portion (32) is inscribed within a respective notched proximal angular sector (33) or separation proximal angular sector (34) with an identical measurement within 5°.

4. The transpedicular anchoring screw (1) according to claim 3, wherein the measurement of each of said notched proximal angular sectors (33) or separation proximal angular sectors (34) is comprised between 30° and 75°.

5. The transpedicular anchoring screw (1) according to any one of claims 1 to 4, wherein at least one of the proximal notches (3) is delimited by a single notch edge (37) shaped as a concave circle arc.

6. The transpedicular anchoring screw (1) according to any one of claims 1 to 5, wherein at least one of the proximal notches (3) is delimited by a notch edge (35) shaped as a convex circle arc.

7. The transpedicular anchoring screw (1) according to any one of claims 1 to 6, wherein the proximal portion (22) and the distal portion (23) are contiguous, one of the separation proximal portions (32) of the proximal portion (22) being contiguous to one of the notched distal portions (41) of the distal portion (23).

8. The transpedicular anchoring screw (1) according to any one of claims 1 to 7, wherein each of the separation distal portions (42) is inscribed within a separation distal angular sector (44) whose measurement is equal to 0°, so that the successive distal notches (4) are contiguous.

9. The transpedicular anchoring screw (1) according to any one of claims 1 to 8, wherein at least one of the distal notches (4) is delimited by a single notch edge (45) shaped as a concave circle arc.

10. The transpedicular anchoring screw (1) according to any one of claims 1 to 9, wherein each notched distal portion (41) is inscribed within a notched distal angular sector (43) whose measurement is comprised between 50° and 75°.

11. The transpedicular anchoring screw (1) according to any one of claims 1 to 10, wherein the notched proximal portions (31) and the separation proximal portions (32) of the proximal portion (22) have a thickness (E1), measured along the screw axis (111), larger than a thickness (E2) of the notched distal portions (41) and of the separation distal portions (42) of the distal portion (23).

12. The transpedicular anchoring screw (1) according to any one of claims 1 to 11, wherein the proximal portion (22) and the distal portion (23) of the first thread (2) extend respectively, along the screw axis (111), over a proximal length and a distal length, said proximal length having an measurement comprised between 0.5 and 1.5 times that of said distal length.

13. The transpedicular anchoring screw (1) according to any one of claims 1 to 12, wherein the screw body (11) has a transverse dimension (D1, D2), measured perpendicularly to the screw axis (111), whose measurement progressively decreases between the proximal end (112) and the distal end (113).

14. The transpedicular anchoring screw (1) according to any one of claims 1 to 13, including a screw head (12) positioned on the proximal end (112) of the screw body (11), said screw head (12) having a collar (7) delimited by a convex-shaped collar edge (71) and having head notches (72) formed in said collar edge (71),
said collar (7) having a transverse dimension (D3), measured perpendicularly to the screw axis (111), larger than a transverse dimension (D1, D2) of the first thread (2), also measured perpendicularly to the screw axis (111).

15. The transpedicular anchoring screw (1) according to any one of claims 1 to 14, wherein at least one amongst a proximal notch (3) and a distal notch (4) is at least partially filled with a bioresorbable material.

16. The transpedicular anchoring screw (1) according to claim 15, wherein each of the proximal notches (3) and each of the distal notches (4) is at least partially filled with the bioresorbable material.

17. The transpedicular anchoring screw (1) according to any one of claims 15 and 16, wherein the bioresorbable material is a polymer, for example a PLA (polyactic acid) type polymer, a PLGA (polyglycolic acid) type polymer or a PLA-PLGA type copolymer.
